Europäisches Patentamt

European Patent Office

Office européen des brevets

(19)

(11) Veröffentlichungsnummer : **0 008 716**
**B1**

(12) **EUROPÄISCHE PATENTSCHRIFT**

(45) Veröffentlichungstag der Patentschrift :
17.03.82

(51) Int. Cl.³ : **C 07 D201/04**

(21) Anmeldenummer : 79102969.7

(22) Anmeldetag : 16.08.79
**Die Akte enthält technische Angaben, die nach dem
Eingang der Anmeldung eingereicht wurden und die
nicht in dieser Patentschrift enthalten sind.**

(54) **Verfahren zur Herstellung von Epsilon-Caprolactam.**

(30) Priorität : 30.08.78 DE 2837793

(43) Veröffentlichungstag der Anmeldung :
19.03.80 (Patentblatt 80/06)

(45) Bekanntmachung des Hinweises auf die Patenterteilung : 17.03.82 Patentblatt 82/11

(84) Benannte Vertragsstaaten :
**BE CH DE FR GB IT NL**

(56) Entgegenhaltungen :
DE - A - 1 953 153
DE - A - 1 955 559
DE - A - 2 003 460
DE - B - 1 227 028
DE - C - 752 574

**Chemical Engineers' Handbook — Fifth Edition —
R.H. Perry — « Uses of fluidized bed » S. 20-73**

(73) Patentinhaber : **BASF Aktiengesellschaft
Carl-Bosch-Strasse 38
D-6700 Ludwigshafen (DE)**

(72) Erfinder : **Fuchs, Hugo, Dr.
Egellstrasse 28
D-6700 Ludwigshafen (DE)**
Erfinder : **Brand, Uwe
Rheingoldstrasse 12
D-6841 Rosengarten (DE)**
Erfinder : **Grosskinsky, Otto-Alfred, Dr.
Semmelweisstrasse 8
D-6700 Ludwigshafen (DE)**
Erfinder : **Schmitz, Ruediger
Pierstrasse 4
D-6710 Frankenthal (DE)**

Imprimerie Jouve, 18, rue St-Denis, 75001 Paris, France

## Verfahren zur Herstellung von ε-Caprolactam

Gegenstand der Erfindung ist ein Verfahren zur Herstellung von ε-Caprolactam durch Umlagerung von Cyclohexanonoxim in der Gasphase bei einer Temperatur von 230 bis 400 °C an Bortrioxid enthaltenden Trägerkatalysatoren im Wirbelbett unter Zuführung von flüssigem Cyclohexanonoxim sowie Inertgasen mit erhöhter Temperatur.

Bei der katalytischen Umlagerung von Cyclohexanonoxim zu ε-Caprolactam bei erhöhter Temperatur an Borsäure, z.B. Boroxid enthaltenden Trägerkatalysatoren in der Wirbelschicht ist eines der Probleme die entstehende Umlagerungswärme abzuführen. Zur Einhaltung der für die Umlagerung günstigsten Reaktionstemperatur von etwa 210-450 °C, muß man daher kühlen, um die freiwerdende Reaktionswärme abzuführen. Diese Kühlung kann über die Wandung des Reaktors oder über in die Wirbelschicht eintauchende Kühlregister erfolgen. Eine Wärmeübertragung durch die Wände ist in so fern von Nachteil, als bei zunehmender Vergrößerung des Reaktors das Volumen mit der 3., die Fläche jedoch nur mit der 2. Potenz zunimmt, so daß die erforderlichen Flächen nicht zur Verfügung stehen. Die Verwendung von Kühlregistern in der Wirbelschicht führt zu Störungen. Dabei kommt es zu unvollständigen Umsetzungen oder auch zu unerwünschten Ablagerungen an den Kühlflächen. Bei dem aus der DE-PS 2 003 460 bekannten Verfahren wird zur Abführung der Umlagerungswärme ein Lösungsmittel mit in die Wirbelschicht eingeführt und durch Verdampfen des Lösungsmittels die Reaktionswärme abgeführt. Diese Arbeitsweise hat jedoch den Nachteil, daß die Lösungsmittel zurückgewonnen und vor ihrer Wiederverwendung gereinigt werden müssen. Selbst wenn man als Lösungsmittel Caprolactam selbst verwendet, ist dies nicht problemlos, da sich bei den hohen angewandten Temperaturen zusätzlich in geringem, jedoch messbaren Ausmaß, Zersetzungsprodukte des Caprolactams bilden. Darüber hinaus wird durch die mitgeführte Menge an Lösungsmitteln, bzw. zusätzlichem Caprolactam die Abscheidung derselben aus dem zur Wirbelung benutzten Inertgasstrom technisch aufwendiger.

Es war deshalb die technische Aufgabe gestellt, bei der katalytischen Umlagerung von Cyclohexanonoxim zu ε-Caprolactam die entstehende Reaktionswärme auf einfachere Weise abzuführen, wobei eine Anpassung an verschiedene Betriebszustände ohne technischen Aufwand möglich ist und die Abscheidung des ε-Caprolactam möglichst einfach gestaltet werden kann.

Diese technische Aufgabe wird gelöst in einem Verfahren zur Herstellung von ε-Caprolactam durch Umlagerung von Cyclohexanonoxim in der Gasphase, bei einer Temperatur von 230 bis 400 °C an Bortrioxid enthaltenden Trägerkatalysatoren im Wirbelbett unter Zuführung von flüssigem Cyclohexanonoxim sowie Inertgas mit erhöhter Temperatur, wobei man in die Wirbelschicht Inertgase mit einer Temperatur von 80 bis 300 °C zuführt und die Umlagerungswärme durch Erhitzen des Inertgases abführt.

Das neue Verfahren hat den Vorteil, daß in der Wirbelschicht keine Kühlflächen angewandt werden müssen. Weiter hat das neue Verfahren den Vorteil, daß keine Lösungsmittel angewandt werden, die zurückgewonnen werden müssen oder die Abscheidevorrichtung belasten. Ferner hat das neue Verfahren den Vorteil, daß die Reaktionstemperatur auf einfache Weise geregelt werden kann, und die Wärmeabfuhr ohne großen Aufwand auf verschiedene Betriebszustände, z.B. geringeren Durchsatz, angepaßt werden kann.

Die Umlagerungsbedingungen für Cyclohexanonoxim zu ε-Caprolactam sind an sich bekannt. Das Cyclohexanonoxim wird zweckmäßig wasserhaltig, z.B. mit bis zu 10 % Wassergehalt angewandt. In der Regel beträgt der Wassergehalt von 3 bis 7 Gewichtsprozent. Das Cyclohexanonoxim wird erfindungsgemäß im flüssigen Zustand der Reaktion zugeführt. Vorteilhaft hat das flüssige Cyclohexanonoxim eine Temperatur von 80 bis 120 °C. In der Wirbelschicht verdampft das Cyclohexanonoxim, in dem Maße wie es eingebracht wird, und lagert in Gegenwart der angewandten Katalysatoren zu ε-Caprolactam um. Vorteilhaft führt man das geschmolzene Cyclohexanonoxim nicht an einer Stelle, sondern an mehreren Stellen, z.B. 2 bis 20 Stellen zu um eine möglichst gleichmäßige Beaufschlagung der Wirbelschicht zu erzielen. Die Anzahl der Zufuhrstellen ist von der Größe des Reaktors abhängig. Die Umlagerung wird bei einer Temperatur von 230 bis 400 °C, vorzugsweise von 270 bis 370 °C durchgeführt. In der Regel führt man die Umsetzung unter Drücken von 1,1 bis 2 bar durch. Man kann jedoch auch bei niedrigeren, als auch höheren Drücken arbeiten.

Als Katalysatoren verwendet man Bortrioxid enthaltende Trägerkatalysatoren. Geeignete Träger sind insbesondere Aluminiumoxid in seinen verschiedenen Modifikationen, wie Tonerde, γ-Aluminiumoxid oder Böhmit. Geeignete Träger sind auch Kieselsäure oder Titandioxid oder Gemische der genannten Oxide untereinander, z.B. Aluminiumsilikate. Das Gewichtsverhältnis von Bortrioxid zu Träger beträgt im allgemeinen von 1 : 9 bis 1: 1. In den bevorzugt verwendeten Katalysatoren beträgt der Anteil an Bortrioxid 25 bis 50 Gewichtsprozent. Die Katalysatoren können darüber hinaus Zusätze, z.B. von Mangan, Kobalt oder Nickelsalzen in Form von Oxid in einer Menge bis zu 10 Gewichtsprozent, bezogen auf den Gesamtkatalysator haben. Die Katalysatoren werden z.B. hergestellt in dem man die Träger mit Borsäure oder solche bildende Verbindungen, wie Ammoniumborat tränkt, bei 50-580 °C trocknet und anschließend zur Umwandlung der aufgebrachten Salze in die entsprechenden Mischphasen mit Boroxid bei 600-1 200 °C glüht. Die Katalysatoren werden in üblicher Weise geformt, z.B. durch Anteigen von Katalysator und Träger mit wenig Wasser, Vermengen und Pressen der Masse zu Strängen oder Pillen. Zweckmäßig verwendet man für die Wirbelschicht Korngrößen von 0,05 bis 1,5 mm, insbesondere 0,2 bis 1,0 mm. Die Höhen der Katalysatorschicht werden zweckmäßigerweise so gewählt, daß die Verweilzeiten des Oxim in der Katalysatorschicht zwischen 0,01 und 30 sec., insbesondere 0,1 bis 5 sec. betragen.

Die Katalysatoren werden als auf- und abwirbelnde Schicht angewandt. Hierzu wird von unten, z.B. an mehreren Stellen, Inertgas in die Schicht eingeführt. Geeignete Inertgase sind beispielsweise Kohlendioxid, Argon oder Stickstoff. Auf Grund der leichten Zugänglichkeit wird Stickstoff bevorzugt. Es versteht sich, daß vor Inbetriebnahme die Wirbelschicht z.B. von außen durch elektrische Heizung auf die gewünschten Reaktionstemperaturen gebracht wird. Ebenfalls ist das Aufheizen dadurch möglich, daß man das Inertgas zunächst auf so hohe Temperaturen bringt, daß das Wirbelgut die erforderliche Reaktionstemperatur erhält.

Erfindungsgemäß führt man Inertgas nach Beginn der Umlagerungsreaktion mit einer Temperatur von 80 bis 300 °C der Wirbelschicht zu. Es versteht sich, daß die Temperatur des zugeführten Inertgases niedriger ist als die jeweils angewandte Reaktionstemperatur. Das zugeführte Gas hält nicht nur den Katalysator in auf- und abwirbelnder Bewegung, sondern nimmt die entstehende Umlagerungswärme auf und führt diese, sowie das gebildete ε-Caprolactam ab. Durch die Wahl der Temperatur sowie der Menge des zugeführten Inertgases ist es möglich die Reaktionstemperatur in der Wirbelschicht auf einfache Weise zu steuern, auch bei wechselnden Mengen an zugeführten Cyclohexanonoxim. Wird beispielsweise weniger Cyclohexanonoxim zuführt, so wählt man eine innerhalb des Bereiches höhere Temperatur, während bei größeren Mengen an Cyclohexanonoxim Temperaturen im unteren Teil des angegebenen Bereiches angewandt werden.

Anschließend kondensiert man das ε-Caprolactam vorteilhaft in einer Kolonne im Gegenstrom, in dem man auf den Kopf der Kolonne Wasser aufgibt und so durch Verdampfen des Wassers die Kondensationswärme abführt. Aus dem über dem Kopf der Kolonne abgehenden Gasstrom entfernt man durch Kühlen die restliche Menge an Wasser und gegebenenfalls Verunreinigungen und führt den Inertgasstrom nach Einstellen der gewünschten Temperatur weiter der Wirbelschicht zu.

Das erfindungsgemäß hergestellte Caprolactam wird für die Herstellung von Polycaprolactam verwendet. Das Verfahren nach der Erfindung sei im folgenden Beispiel veranschaulicht.

Beispiel

In einem Reaktor von 5 000 mm Länge und 1 200 mm Durchmesser mit Zuführungen für geschmolzenes Cyclohexanonoxim an 4 Stellen und einer Zuführung für Inertgas unten werden 500 kg Katalysator bestehend aus einem bei 800 °C geglühten Gemisch von Aluminiumoxid mit einem Gehalt von 50 Gewichtsprozent Boroxid und einer Korngröße von 0,5 bis 1,0 mm angeordnet. Durch einen von unten eingeblasenen, zunächst auf 360 °C erwärmten Stickstoffstrom von 650 kg pro Stunde, wird der Katalysator aufgeheizt und zum Wirbeln gebracht. Nun werden stündlich 400 kg Cyclohexanonoxim flüssig zudosiert und gleichzeitig die Temperatur des zugeführten Stickstoffstromes auf 130 °C gesenkt. Während der Reaktion werden stündlich ca. 200 kg Frischkatalysator in die Wirbelschicht eingebracht und die entsprechende Menge verbrauchten Katalysators abgezogen. Aus den Dämpfen die den Reaktor verlässt wird das ε-Caprolactam in einer Glockenbodenkolonne mit 10 Böden durch Aufgabe von 90 kg Wasser auf dem Kolonnenkopf kondensiert und in einem nachgeschalteten Kühler Wasser und Verunreinigungen aus dem Stickstoffstrom abgeschieden. Der Stickstoff wird nach Aufheizen auf 130 °C wieder der Wirbelschicht zugeführt. Die Ausbeute an ε-Caprolactam beträgt 95 %.

Ein analoges Ergebnis erhält man, wenn man der Wirbelschicht stündlich 200 kg Cyclohexanonoxim von 90 °C zuführt und 650 kg Stickstoff von 260 °C in die Wirbelschicht einführt.

## Anspruch

Verfahren zur Herstellung von ε-Caprolactam durch Umlagerung von Cyclohexanonoxim in der Gasphase bei einer Temperatur von 230 bis 400 °C an Bortrioxid enthaltenden Trägerkatalysatoren im Wirbelbett unter Zuführung von flüssigem Cyclohexanonoxim, sowie Inertgas mit erhöhter Temperatur, dadurch gekennzeichnet, daß man Inertgas mit einer Temperatur von 80-300 °C zuführt und die Umlagerungswärme durch Erhitzen des Inertgases abführt.

## Claim

A process for the preparation of ε-caprolactam by rearranging cyclohexanone-oxime in the gas phase at from 230 to 400 °C over a supported catalyst, containing boron trioxide, in a fluidized bed, whilst feeding in liquid cyclohexanone-oxime and inert gas at elevated temperature, characterized in that inert gas is fed in at a temperature of from 80 to 300 °C and the heat of rearrangement is removed by a rise in temperature of the inert gas.

## Revendication

Procédé de préparation d'ε-caprolactame par transposition de cyclohexanonoxime en phase gazeuse, à une température de 230 à 400 °C, sur des catalyseurs à support contenant du trioxyde de bore, en lit turbulent, avec amenée de cyclohexanonoxime ainsi que de gaz inerte à température élevée, caractérisé par le fait que l'on amène du gaz inerte à une température de 80-300 °C et l'on évacue la chaleur de transposition par chauffage du gaz inerte.